# EUROPEAN PATENT APPLICATION

(11) **EP 4 390 412 A1**
(43) Date of publication of application: **26.06.2024**
(21) Application number: 22215871.9
(22) Date of filing: 22.12.2022
(51) Int. Cl.: G01R 31/12

(54) **ARC DETECTION**

(71) Applicant: Koninklijke Philips N.V., 5656 AG Eindhoven (NL)
(72) Inventor: AGAFONOV, Aleksei, Eindhoven (NL); VERNICKEL, Peter, Eindhoven (NL); IMMINK, Albert Hendrik Jan, 5656AG Eindhoven (NL); WEISS, Steffen, Eindhoven (NL); BASTIAANSEN, Thomas, Eindhoven (NL); LEUSSLER, Christoph Günther, Eindhoven (NL); LIPS, Oliver, Eindhoven (NL); VOGTMEIER, Gereon, Eindhoven (NL); RIBBING, Carolina Maria, Eindhoven (NL)
(74) Representative: Philips Intellectual Property & Standards

(57) **Abstract**

An apparatus (30) for detecting an electric arc (24a, 24b, 24c) in an electrical system (10) is provided. The apparatus facilitates localizing the position of the electric arc e.g., on component level. The apparatus comprises a sensor interface circuit (32) and one or more processors (34). The sensor interface circuit is configured to receive a signal originating from at least one sensor that is arranged in one or more positions in the electrical system for detecting an electrical current. The one or more processors are configured to analyze the received signal to determine a direction of the electrical current at the one or more positions in the electrical system, and to determine, based on the direction of the electrical current, an origin of the electric arc in the electrical system.

## Description

### FIELD OF THE INVENTION

One aspect of the present disclosure relates to an apparatus for detecting an electric arc in an electrical system. A system, a method, and a sensor kit for use in detecting an electric arc in an electrical system, are also disclosed. Another aspect of the present disclosure relates to a monitoring apparatus for determining an origin of an arcing event in an X-ray imaging system.

### BACKGROUND OF THE INVENTION

The high voltage component chain in electrical systems such as medical imaging systems includes active and passive components. For example, a so-called "generator-cable-tube" system used in an X-ray imaging system comprises a high-voltage "HV" generator, one or more cables, and an X-ray tube. Over time, the components in such systems can degrade, and this degradation can give rise to electrical arcing in the components. After an arc has occurred, it is important to identify the component in which the arc has occurred. In some cases, the performance of the component may require verification. In other cases, a service operation, or repair operation may need to be performed on the component. For instance, the functionality of the component may need to be verified and/or the component may need to be repaired or replaced. In one known solution to this issue, a component in which an arc has occurred is identified by equipping components with expensive monitoring equipment that measure currents and voltages at high temporal resolution.

### SUMMARY OF THE INVENTION

There may be a need to locate the origin of an electric arc in an electrical system, e.g. at a component level.

This need is addressed by the subject-matter of the independent claims. Further examples are incorporated in the dependent claims. It should be noted that the following described aspects of the disclosure apply equally to the apparatus, the system, to the method, and to the sensor kit for use in detecting an electric arc in an electrical system.

According to a first aspect of the present disclosure, there is provided an apparatus for detecting an electric arc in an electrical system. The apparatus comprises a sensor interface circuit and one or more processors. The sensor interface circuit is configured to receive a signal originating from at least one sensor that is arranged in one or more positions in the electrical system for detecting an electrical current. The one or more processors are configured to analyze the received signal to determine a direction of the electrical current at the one or more positions in the electrical system, and to determine, based on the direction of the electrical current, an origin of the electric arc in the electrical system.

As discussed above, the ability to detect arcing is an important aspect of the monitoring of electrical systems, such as medical imaging systems, for example. In an X-ray imaging system, for instance, of the ability to localize an electric arc within the HV component chain is important since it enables e.g. the functionality of the component to be verified, or the component to be replaced, in a time-efficient manner. However, a first-time-right identification of arcing components within an X-ray generation chain is not straightforward. Consequently, in practice, components in an X-ray imaging system that might be responsible for arcing, such as the X-ray tube and the HV generator, are often replaced sequentially until arcing no longer occurs. Afterwards, any parts that have been replaced unnecessarily may be removed, which is time-consuming, or they may simply be left in the X-ray imaging system in order to save time.

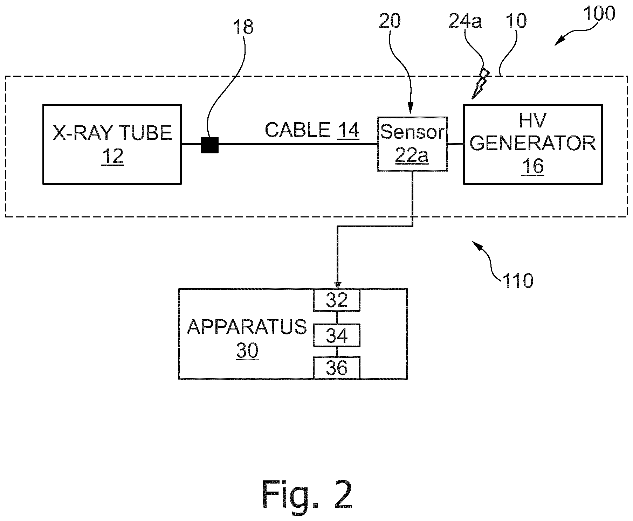

To that end, the present disclosure proposes an apparatus, a system, and a method to determine an origin of an electric arc by determining a direction of the electrical current at the one or more positions in the electrical system. The origin of the arc may be determined as a location in the electrical system relative to the one or more positions in the electrical system. The origin may be identified as a component within the electrical system, for example. The direction of the electrical current may be determined in a straightforward manner, and this gives rise to several advantages over the known use of expensive measurement equipment. In some examples it is deemed sufficient to detect whether the electrical current at one or more positions in the electrical system has temporarily reversed. This detection may also be performed during normal operation of the electrical system. One or more low-cost detectors may be sufficient to localize the component in which an arc has occurred. Thus, it facilitates the verification of the arcing component, and where necessary, its repair or replacement, in a time-efficient manner, and also obviates the complexity of known arc detection systems. The apparatus will be described with reference to the examples shown in Figs. 2, 4 and 5.

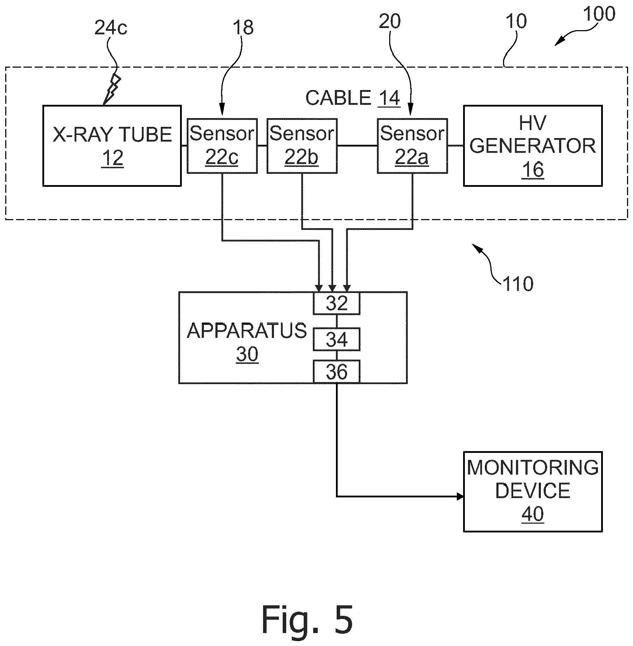

According to an example of the present disclosure, the one or more processors are configured to analyze the received signal to determine a magnitude of the electrical current at one or more positions in the electrical system. The one or more processors are configured to determine the origin of the electric arc in the electrical system based further on the magnitude of the electrical current. In some cases, the magnitude of the change in electrical current provides information that can be used in addition to the sign of the electrical current to determine the origin of a component in which an electric arc has occurred. For instance, in a network of electrical components, multiple sensors may detect that an arc has occurred based on the sign of the electrical current. Additional information that indicates the magnitude of the electrical current that is detected by each of the sensors, may be used to determine the hierarchical position of the arcing component within a network of electrical components, and thereby localize the position of the arc with greater accuracy. This will be explained hereinafter and in particular with respect to the example shown in Fig. 5.

According to an example of the present disclosure, the one or more processors are configured to determine a time period during which the electrical current has reversed its direction at the one or more positions in the electrical system during an operation of the electrical system, to determine an integral of the electrical current over the determined time period, and to determine an arc strength of the electric arc based on the determined integral. The one or more processors are configured to determine the origin of the electric arc in the electrical system based further on the magnitude of the electrical current.

*The integral of the reversed current, i.e., discharging current, can be tracked as a measure of arc strength, which can be related to the damage that the electric arc caused. Potentially, it can also give additional clues to where the electric arc happened more precisely if tube voltage and stray capacitances are known. This would be valuable e.g. for discrimination between arcing in the cathode side HV connector versus arcing cathode-GND in the tube vacuum for an X-ray imaging system.*

According to an example of the present disclosure, the one or more processors are configured to compare the measured electrical current with a defined threshold to determine a severity of the electric arc.

With the known stray capacitance of tube and cables, system-specific thresholds for reverse currents may be set, and straightforward circuits, such as comparator or trigger circuits, may be used to detect whether these thresholds have been exceeded, indicating the severity of the electric arc. For example, the severity of the electric arc may include *minor, medium, and strong.*

According to an example of the present disclosure, in response to determining that the severity of the electric arc exceeds the defined threshold, the one or more processors are configured to trigger an action to mitigate the severity of the electric arc.

*For example, severe events may be used to trigger actions to mitigate the severity of the electric circ, such as inhibiting tube operation.*

According to an example of the present disclosure, in response to determining that the severity of the electric arc is below the defined threshold, the one or more processors are configured to record information relating to the electric arc.

*Minor arcing events, for instance, arcing events that are not immediately detrimental to the X-ray tube performance, may allow continued operation of a component. Such arcing events may simply be counted in order to collect statistical information on arcing. Binning the detected arcing events into two or more types, e.g., minor, medium, and strong, depending on the* magnitude of the change in electrical current *in relation to defined thresholds may be performed in order to provide such statistical information. Correlation of these statistical data with operation modes may provide clues on the root cause of an electric arc, and therefore how arcing may be prevented in future.*

According to an example of the present disclosure, the sensor interface circuit is configured to receive at least two signals originating from at least two sensors that are arranged in at least two positions in the electrical system for detecting the electrical current. The one or more processors are configured to determine the origin of the electric arc by comparing the at least two received signals.

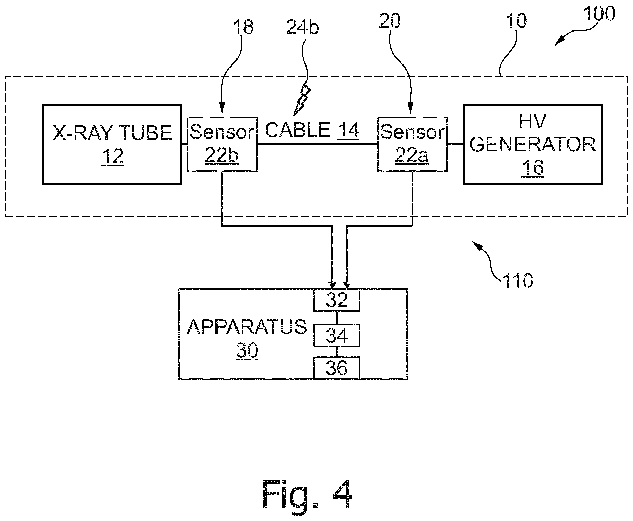

This will be explained hereinafter and in particular with respect to the examples shown in Fig. 4 and 5.

According to an example of the present disclosure, the apparatus further comprises an output circuit. The one or more processors are configured to provide information relating to the electric arc via the output circuit.

According to an example of the present disclosure, the electrical system comprises an X-ray tube, a high-voltage generator, and a cable connecting the X-ray tube and the high-voltage generator.

According to an example of the present disclosure, the electrical system comprises a high-power transmitting radio device.

According to a second aspect of the present disclosure, there is provided a system for detecting an electric arc in an electrical system, the system comprising:
- at least one sensor; and
- an apparatus according to any one of the preceding claims;

wherein the at least one sensor is removably mountable at one or more positions in an electrical system for detecting an electrical current in the electrical system; and
wherein the apparatus is configured to receive a signal originating from the at least one sensor and to detect an electric arc in the electrical system.

This will be explained in detail hereinafter and in particular with respect to the examples shown in Figs. 2, 4 and 5.

According to an example of the present disclosure, the at least one sensor comprises a current sign detector for detecting a direction of the electrical current.

Examples of the electrical current sign detector may include, but are not limited to, a resistor, a capacitively or inductively coupled component, and a Hall sensor.

According to an example of the present disclosure, the system further comprises a monitoring device configured to provide a local or remote analysis of information relating to the electric arc provided by the apparatus.

In some examples, the monitoring device may be a local workstation or server.

In some other examples, the monitoring device may be a cloud-based service provider. According to a third aspect of the present disclosure, there is provided a method for detecting an electric arc in an electrical system, the method comprising:
- receiving a signal originating from at least one sensor that is arranged in one or more positions in the electrical system for detecting an electrical current;
- analyzing the received signal to determine a direction of the electrical current at the one or more positions in the electrical system; and
- determining, based on the direction of the electrical current, an origin of the electric arc in the electrical system.

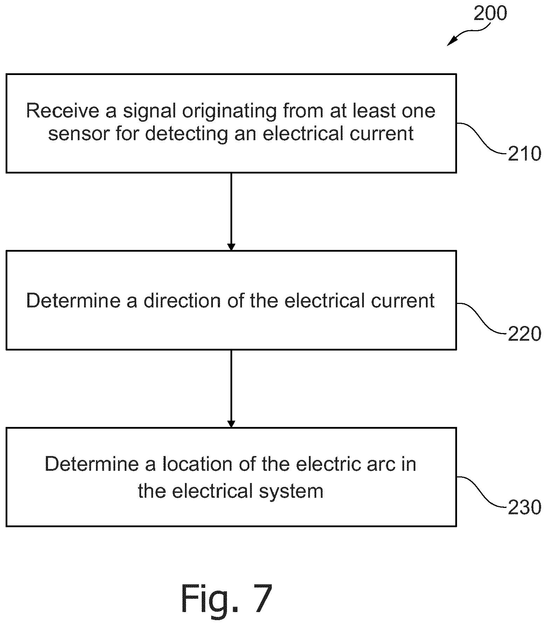

This will be explained in detail hereinafter and in particular with respect to the example shown in Fig. 7.

According to a further aspect of the present disclosure, there is provided a sensor kit for use in detecting an electric arc in an electrical system, the sensor kit comprising a plurality of sensors for detecting an electrical current.

In some examples, the sensor kit may comprise one or more sensors configured to measure a direction of the electrical current.

In some examples, the sensor kit may comprise one or more sensors configured to measure a magnitude of the electrical current.

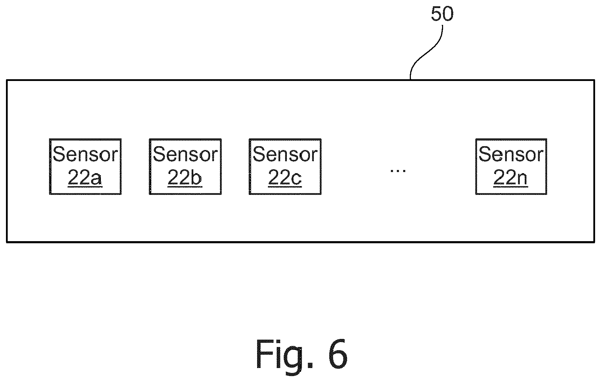

This will be explained in detail hereinafter and in particular with respect to the example shown in Fig. 6.

According to another aspect, there is provided a computer program for controlling an apparatus according to the first aspect, which when executed by a processor is configured to carry out the method according to the third aspect, and/or a computer program for controlling a system according to the second aspect, which when executed by a processor is configured to carry out the method of the fourth aspect.

According to another aspect, there is provided a computer readable medium having stored the program element.

Another aspect of the present disclosure relates to a monitoring apparatus for determining an origin of an arcing event in an X-ray imaging system. Arcing events can occur in different components of an X-ray imaging system, as described above. For instance, arcing events may occur in the voltage generator, in the X-ray tube, or in a cable coupling the X-ray tube to the generator. Arcing events can also occur within different media in such components. For instance, arcing events may occur within media such as vacuum, air, cooling fluid, oil, vacuum, in such components. Arcing events also emit energy in the form of different types of radiation. For instance, arcing events may emit energy in the form of radiofrequency "RF" radiation, (ultra)sonic radiation, and optical radiation. Some of these types of emitted radiation, such as acoustic radiation, can also give rise to vibration when they interact with matter. The inventors have observed that the medium in which an arcing event occurs, and also any shielding that may surround the medium, affect the amount of the different types of radiation that may be detected following an arcing event. Thus, the origin of an arcing event may be determined by detecting different types of radiation that are emitted by an arcing event.

In this regard, a monitoring apparatus 60 for determining an origin of an arcing event 24d in an X-ray imaging system comprising an X-ray tube 12 having a vacuum-containing envelope 62, a liquid cooling circuit 64 for cooling the X-ray tube, and voltage generator 16 configured to power the X-ray tube, is provided. The monitoring apparatus 60 comprises:
a plurality of sensors 66₁, 66₂; and
one or more processors 34;
wherein the plurality of sensors comprises a first sensor 66₁ configured to detect radiofrequency, RF, radiation generated by the arcing event, and a second sensor 66₂ configured to detect vibration and/or acoustic radiation generated by the arcing event; and
wherein the one or more processors 34 are configured to:
   receive signals generated by the first sensor 66₁ and the second sensor 66₂ in response to the detection of the arcing event; and
   selectively identify the origin of the arcing event as one of: within the vacuum-containing envelope 62, within the liquid cooling circuit 64, and within the voltage generator 16, based on the received signals.

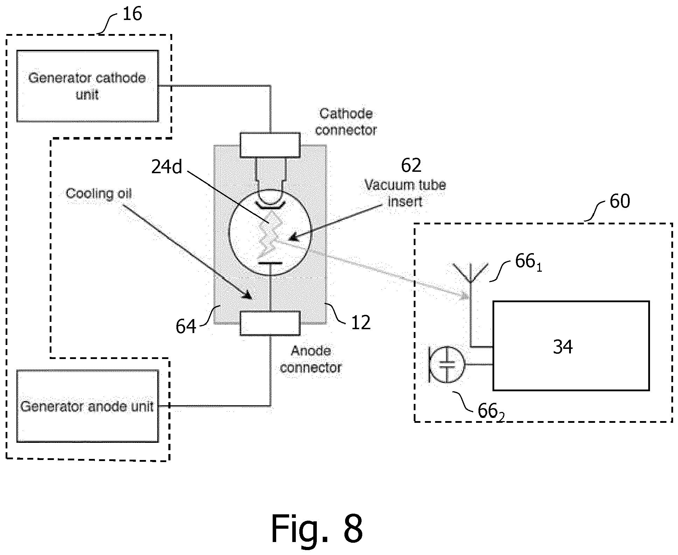

This aspect is described with reference to Fig. 8 - Fig. 11, and is based on the observation that signals that the origin of an arcing event may be assigned to one of: within the vacuum-containing envelope 62, within the liquid cooling circuit 64, and within the voltage generator 16, based on the detection of RF radiation generated by the arcing event, and vibration and/or acoustic radiation generated by the arcing event. For instance, an arc that has been generated within the vacuum-containing envelope 62, typically generates relatively strongly-detectable RF radiation, and relatively weakly-detectable, or undetectable, vibration and/or acoustic radiation. By contrast, an arc that has been generated within the liquid cooling circuit 64, typically generates relatively strongly-detectable RF radiation as well as relatively strongly-detectable vibration and/or acoustic radiation. By contrast, an arc that has been generated within the voltage generator typically generates relatively weakly-detectable, or undetectable, RF radiation, and relatively strongly-detectable vibration and/or acoustic radiation. As described in more detail below, this is due to the inability of the vacuum-containing envelope 62 to transmit vibration and/or acoustic radiation, and the high degree of RF shielding provided by the RF generator, and which consequently reduce the transmission of vibration and/or acoustic radiation, and RF radiation, respectively.

The ability to determine the origin of an arcing event facilitates the verification, repair, or replacement of the affected component in a time-efficient manner, as described above. Moreover, the ability to distinguish the origin of an arcing event between the vacuum-containing envelope, the liquid cooling circuit, and the voltage generator may facilitate a repair of a component rather than its entire replacement. For instance if the origin of an arcing event is determined to be within the liquid cooling circuit, the cooling liquid may be replaced rather than replacing the entire X-ray tube. Likewise, the origin of an arcing event is determined to be within the vacuum-containing envelope, a repair may be possible by "re-conditioning" the X-ray tube, or performing a "tube degassing" procedure on the X-ray tube, rather than replacing it.

In a related example, the one or more processors 34 of the monitoring apparatus 60 are further configured to:
perform a spectral analysis on the signals generated by the first sensor 66₁ and the second sensor 66₂ to generate corresponding first and second frequency spectra;
determine a similarity between each of the first and second frequency spectra and one or more reference frequency spectra, the reference frequency spectra representing signals generated within one or more of: the vacuum-containing envelope 62, the liquid cooling circuit 64, and the voltage generator 16, by the first sensor 66₁ and the second sensor 66₂, respectively; and
wherein the one or more processors 34 are configured to selectively identify the origin of the arcing event, based further on the similarity.

This example is based on the observation that RF radiation generated by the arcing event, and vibration and/or acoustic radiation generated by the arcing event, have characteristic signatures that may be used to identify their origin. For instance, the RF spectrum generated in response to an arcing event within the vacuum-containing envelope 62 is different to the RF spectrum generated in response to an arcing event within the liquid cooling circuit 64. Thus, by performing a spectral analysis on the signals generated by the first sensor 66₁ and the second sensor 66₂ to generate corresponding first and second frequency spectra, the frequency spectra may be compared with reference frequency spectra that are characteristic of signals generated by the first sensor 66₁ and the second sensor 66₁, respectively. The reference frequency spectra may be generated within e.g. the vacuum-containing envelope 62, the liquid cooling circuit 64, and the voltage generator 16. If the detected spectrum is similar to a reference spectrum, the origin of the reference spectrum may be assigned to the detected spectrum. This provides increased confidence in the origin of the arcing event.

In another related example, the plurality of sensors of the monitoring apparatus 60 comprises a third sensor 66₃ configured to detect radiofrequency, RF, radiation generated by the arcing event, and a fourth sensor 66₄ configured to detect vibration and/or acoustic radiation generated by the arcing event. In this example, the one or more processors 34 are further configured to:
receive signals generated by the third sensor 66₃ and the fourth sensor 66₄ in response to the detection of the arcing event; and
estimate a position of the origin of the arcing event based on the detected RF radiation generated by the arcing event using the signals generated by the first sensor 66₁ and the third sensor 66₃; and
estimate a position of the origin of the arcing event based on the detected vibration and/or acoustic radiation generated by the arcing event using the signals generated by the second sensor 66₂ and the fourth sensor 66₄; and
wherein the one or more processors 34 are configured to selectively identify the origin of the arcing event based further on the position of the origin of the arcing event estimated based on the detected RF radiation and/or the position of the origin of the arcing event estimated based on the detected vibration and/or acoustic radiation.

In this example, the signals generated by the third sensor 66₃ and the fourth sensor 66₄ are therefore used in combination with the signals generated by the first sensor 66₁ and the second sensor 66₂, respectively, to determine the origin of the arcing event. In this example, the operations of estimating a position of the origin of the arcing event, may in general be performed by comparing the times, or the phases, or the magnitudes, of the signals detected by the first sensor 66₁ and the third sensor 66₃, and likewise comparing the times, or the phases, or the magnitudes, of the signals detected by the second sensor 66₂ and the fourth sensor 66₄. For instance, in one technique the times of the detected signals may be compared in order to determine that the origin of the arcing event is nearer to the sensor that generated the earlier signal in response to the arcing event. Similarly, in another technique, the magnitudes of the detected signals may be compared in order to determine that the origin of the arcing event is nearer to the sensor that generated the signal with the relatively larger magnitude in response to the arcing event. Similarly, in another technique, the phases of the detected signals may be compared in order to determine that the origin of the arcing event is nearer to the sensor that generated the signal with a relatively earlier specified phase angle in response to the arcing event.

In another technique, a triangulation operation is performed. In this technique, the operation of estimating a position of the origin of the arcing event based on the detected RF radiation generated by the arcing event, may include performing a triangulation operation on the signals generated by the first sensor 66₁ and the third sensor 66₃ to provide the estimated position of the origin of the arcing event based on the detected RF radiation. Likewise, the operation of estimating a position of the origin of the arcing event based on the detected RF radiation generated by the arcing event, may include performing a triangulation operation on the signals generated by the second sensor 66₂ and the fourth sensor 66₄ to provide the estimated a position of the origin of the arcing event based on the detected vibration and/or acoustic radiation.

The triangulation may be performed by comparing the times, or the phases, or the magnitudes, of the signals detected by the first sensor 66₁ and the third sensor 66₃, and likewise comparing the times, or the phases, or the magnitudes, of the signals detected by the second sensor 66₂ and the fourth sensor 66₄. By providing two sensors that detect each type of radiation, e.g. RF radiation, or vibration and/or acoustic radiation, in accordance with this technique, the one or more processors may perform a triangulation operation that estimates the position of the origin of the arcing event as a position anywhere along an arc of revolution around an axis connecting the two sensors. By providing three sensors that detect each type of radiation, the one or more processors may perform a triangulation operation that estimates the position of the origin of the arcing event as a point in space. This assumes that the three sensors are arranged in a non-collinear manner. The additional spatial information on the origin of the arcing event that is provided in accordance with this example provides increased confidence in the determined origin.

In another related example, the monitoring apparatus 60 comprises at least one current sensor 68₁, 68₂. The at least one current sensor 68₁, 68₂ is configured to detect an electrical current flowing into or returning from at least one of: the X-ray tube, the voltage generator, and an electrical cable 70₁, 70₂ coupling the X-ray tube to the voltage generator. The one or more processors 34 are further configured to receive signals generated by the at least one current sensor 68₁, 68₂ in response the arcing event. The one or more processors 34 are configured to selectively identify the origin of the arcing event based further on the signals generated by the at least one current sensor 68₁, 68₂.

The current sensor(s) in this example detect electrical currents that flow into, or that return from, one or more of: the X-ray tube, the voltage generator, and the electrical cable 70₁, 70₂. Electrical arcing events can give rise to perturbations (e.g. spikes) in such detected currents, depending on the origin of the arcing event. For instance, an arcing event originating within the vacuum-containing envelope of the X-ray tube can give rise to a relatively larger perturbation in an electrical current flowing into or returning from the X-ray tube, than an electrical arcing event originating within the liquid cooling circuit. Thus, the additional information that is provided by the detected electrical currents may be used to identify the origin of the arcing event with increased confidence. The current sensor may also be used to selectively identify the origin of the arcing event as within the electrical cable 70₁, 70₂, based on the received signals generated by the at least one current sensor 68₁, 68₂. For instance, an arcing event originating within the cable may give rise to a perturbation with a smaller magnitude than an arcing event originating within the X-ray tube.

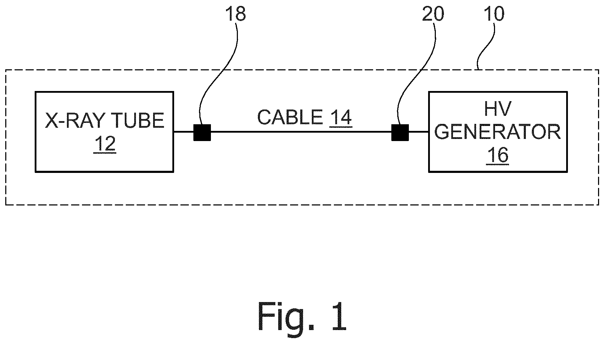

In another related example, the one or more processors 34 of the monitoring apparatus 60 are configured to determine a direction of the electrical current detected by the at least one current sensor 68₁, 68₂. The one or more processors are configured to selectively identify the origin of the arcing event based further on the direction of the electrical current detected by the at least one current sensor 68₁, 68₂. This example operates in the manner described above with reference to Fig. 1 - Fig. 7. In other words, the direction of the electrical current that is generated in response to the arcing event can indicate which side of the current sensor the arcing event occurred.

In another related example the one or more processors 34 of the monitoring apparatus 60 are further configured to automatically generate a service work order 72 based on the received signals generated by the first sensor and/or the second sensor in response to the detection of the arcing event. The service work order comprises information representing the identified origin of the arcing event.

Thus, the service work order enables a service engineer to verify the operation of the vacuum-containing envelope 62, the liquid cooling circuit 64, and the voltage generator 16, as necessary, in a time-efficient manner.

In another related example, an X-ray imaging system that includes the monitoring apparatus 60, described above, is provided.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein.

These and other aspects of the disclosure will be apparent from and elucidated with reference to the example(s) described hereinafter.

### BRIEF DESCRIPTION OF THE DRAWINGS

In the drawings, like reference characters generally refer to the same parts throughout the different views. Also, the drawings are not necessarily to scale, emphasis instead generally being placed upon illustrating the principles of the disclosure.
Fig. 1 illustrates an example of an electrical system.
Fig. 2 illustrates an example of a system according to an example of the present disclosure.
Fig. 3 illustrates a Spice model of an X-ray tube component chain.
Fig. 4 illustrates a further example of a system according to an example of the present disclosure.
Fig. 5 illustrates a still further example of a system according to an example of the present disclosure.
Fig. 6 illustrates an example of a sensor kit for use in detecting an electric arc in an electrical system.
Fig. 7 illustrates a flow chart describing an example of a method for detecting an electric arc in an electrical system.
Fig. 8 is a schematic diagram illustrating a first example of a monitoring apparatus 60 for determining an origin of an arcing event 24d in an X-ray imaging system, in accordance with some aspects of the present disclosure.
Fig. 9 is a schematic diagram illustrating a second example of a monitoring apparatus 60 for determining an origin of an arcing event 24d in an X-ray imaging system, in accordance with some aspects of the present disclosure.
Fig. 10 is a schematic diagram illustrating a third example of a monitoring apparatus 60 for determining an origin of an arcing event 24d in an X-ray imaging system, in accordance with some aspects of the present disclosure.
Fig 11 is a schematic diagram illustrating a fourth example of a monitoring apparatus 60 for determining an origin of an arcing event 24d in an X-ray imaging system, in accordance with some aspects of the present disclosure.

### DETAILED DESCRIPTION OF EMBODIMENTS

In the following description, reference is made to an apparatus for detecting an electric arc in electrical systems. Electrical systems often employ high voltages, i.e. voltages that are greater than 0.1 kV, or greater than 1 kV, or greater than 10 kV, and electrical arcing can occur in such systems due to the deterioration of components in the systems over time. Reference is made to examples in which the electrical systems are medical imaging systems, e.g. an X-ray or a CT imaging system. However, it will be appreciated that the above- and below-described apparatus, method, system, and sensor kit may find use in electrical systems in general. By way of another example, the apparatus, method, system, and sensor kit may find use in high-power transmitting radio devices. In this context, high-power, refers to a poser consumption that is greater than 0.1 kW, greater then 1 kW, or greater than 10 kW.

Fig. 1 illustrates an example of an electrical system 10. In this example, the electrical system 10 is a so-called "generator-cable-tube" system that comprises an X-ray tube 12, a cable 14, and a HV generator 16. The multi-component high-voltage system 10 shown in Fig. 1 may be implemented in an X-ray *imaging* system (not shown). Examples of the X-ray *imaging* system may include, but are not limited to, a projection X-ray imaging system such as an X-ray C-arm-based *imaging* system or a digital X-ray radiography "DXR" *imaging* system, or a computed tomography "CT" *imaging* system. The following discussion of the multi-component high-voltage system 10 is merely an example of such implementation and is not intended to be limiting to a specific type of X-ray imaging system.

The X-ray tube 12 is configured to produce X-rays. In general the X-rays may have an energy, or a range of energies that are within the range from approximately 30 keV to 200 keV. An X-ray tube typically includes an evacuated tube, an anode, and a cathode. In-use, the cathode is heated whilst an electrical field is applied between the anode and the cathode.. The electrical field liberates electrons from the cathode, and accelerates the electrons towards the anode, where they are stopped and emit Bremsstrahlung radiation. An example of a type of the X-ray tube 12 is a glass tube that is surrounded by a (usually, but maybe not necessarily) metal chassis. In this example, the region between the glass tube and the metal chassis may be filled with a liquid cooling medium such as oil.

As shown in Fig. 1, the X-ray tube 12 is removably coupled to the cable 14 at a tube-cable interface 18. The cable 14 is removably coupled to the HV generator 16 at the generator-cable interface 20. The HV generator 16 supplies a high voltage for operating the X-ray tube 12.

Imperfections and aging of the components can give rise to electrical arcing. Arcing may be caused by various effects, such as for example the ionization of residual particles within the glass tube. Electrical arcs disrupt the X-ray generation process. This is detrimental to an imaging examination that relies on a continuous supply of X-rays. Arcing cannot be completely avoided, but reliable detection can help improve maintenance procedures.

For effective service and repair procedures of imaging systems such as the X-ray imaging system described above, it is important to be able to localize the position of the electric arc at a component level. In one known solution to this issue, a component in which an arc has occurred is identified by equipping components in electrical systems with expensive monitoring equipment that measure currents and voltages in the electrical system at high temporal resolution.

In order to overcome the complexity of the known monitoring equipment, the present disclosure proposes a straightforward approach that monitors the direction of the electrical current.

Fig. 2 illustrates an example of a system 10 according to an example of the present disclosure. The example system 100 comprises an electrical system 10 and a system 110 for detecting an electric arc in the electrical system 10. The system 110 comprises a sensor 22 and an apparatus 30 for detecting an electric arc in the electrical system 10.

In the example shown in Fig. 2, the electrical system 10 is a generator-cable-tube system as shown in Fig. 1. Items in Fig. 2 with the same label as in Fig. 1 refer to the same items, and the description of the same item will be not repeated.

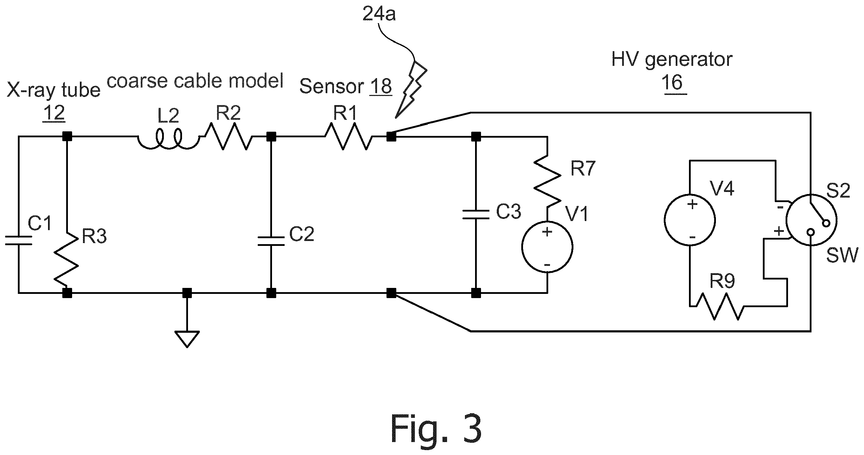

The inventors have found out that during normal operation, the electrical current on the high voltage rails has a specific direction, i.e., sign. This applies to both DC currents as well as AC currents, the sign of the current in the latter being changed twice per cycle during normal operation. Components in an electrical circuit may also have a stray capacitance, which becomes charged over time. An electric arc in an electrical component gives rise to an instantaneous reduction in its impedance. Thus, if arcing occurs during the operation of a component, the source of the current may be incapable of supplying the arcing current. In this case, the capacitance of a source of electrical current start to discharge, leading to a reduction of the voltage across its supply terminals. The discharging current is directed towards the arcing component. Currents flowing in components that are "behind the arc", as seen from the current source will change direction until the electrical arc has ended. This will be explained in detail with respect to the Spice model shown in Fig. 3.

Fig. 3 illustrates a Spice model of an X-ray tube component chain. In this example, it is assumed that an electric arcing event occurs close to the HV generator 16.

The X-ray tube 12, the cable 14, and the HV generator 16 have capacitances and resistances, which may depend on the operational status of the X-ray tube (e.g. a bias voltage that is applied to the X-ray tube). For example, as shown in Fig. 3, the X-ray tube 12 may be modeled as a capacitor C1 and a resistor R3 parallel to the capacitor C1. The cable 14 may be modeled using a coarse cable model and represented by an inductor L2, a resistor R2, and a capacitor C2. The HV generator 16 may be modeled as a voltage generator V1, a resistor R7, and a capacitor C3 parallel to the voltage generator V1.

Capacitances shown in Fig. 3 are charged by the high voltage of the HV generator V1. In-use, the X-ray tube draws a positive current through the chain, which can be detected by the sensor 18, such as a detecting resistor R1 shown in Fig. 3

An electric arc event that occurs close to the HV generator 16, such as electric arc 24a illustrated in Fig. 3, may be simulated by a voltage generator V4, a resistor R9, and a switch S2. It is noted that the voltage generator V4, the resistor R9, and the switch S2 shown in Fig. 3 are illustrated to emulate the electric arc 24a in simulation or descriptive environment, and are thus not physical components of the electrical system 10, e.g., tube-cable-generator system illustrated in Fig. 3, nor the circuitry proposed in the present disclosure. During the arc event, the impedance seen by the HV generator 16 drops instantaneously. The HV generator 16 cannot maintain the high voltage, and consequently stray capacitances discharge. Discharging the capacitors C1 and C2 leads to a negative current through R1.

This change of the sign may be detected by known comparator or trigger circuits. If detector circuits are placed at the interfaces of the components in the electrical system, such as at the tube-cable interface 18, or at the generator-cable interface 20, it becomes possible to identify the origin of the electric arc more accurately.

In another example, turning back to Fig. 2, a sensor 22, such as the sensor 22a illustrated in Fig. 2, is provided and removably mounted at the generator-cable interface 20 to monitor the electrical current. As described hereinbefore, and with reference to the Spice model of an X-ray tube component chain shown in Fig. 3, the X-ray tube operation results in a positive current through the chain, and the electrical current flows to the X-ray tube 12. In case of an electric arc event close to the HV generator 16, such as electric arc 24a shown in Fig. 2, the HV generator 16 is incapable of supplying the current, , and consequently the stray capacitances discharge, which leads to a negative current, and the voltage across the terminals of the HV generator 16 consequently falls. The change of the sign of the current can be detected by the sensor 22a. It is noted that in order to detect the sign of the current, , it is sufficient to detect whether the electrical current between generator and cable has reversed instantaneously during normal operation. With the known stray capacitance of tube and cables, system-specific thresholds for reverse currents may be set, and straightforward circuits, such as comparator or trigger circuits, may be used to detect whether these thresholds have been exceeded, thereby indicating the presence of an electric arc in the vicinity of the detected electrical current. Examples of the sensor 22 may include, but are not limited to, a resistor, a capacitively coupled component, an inductively coupled component, and a Hall sensor.

The sensor 22a may comprise a transmitter configured to transmit sensor data via a signal to the apparatus 30 via a wired (e.g., cable) connection or via a wireless (e.g., Bluetooth) connection. The apparatus 30 shown in Fig. 2 includes an sensor interface circuit 30, one or more processors 34, and an output circuit 36.

In general, the apparatus 30 may comprise various physical and/or logical components for communicating and manipulating information, which may be implemented as hardware components (e.g., computing devices, processors, logic devices), executable computer program instructions (e.g., firmware, software) to be executed by various hardware components, or any combination thereof, as desired for a given set of design parameters or performance constraints. Although Fig. 2 may show a limited number of components by way of example, it can be appreciated that a greater or a fewer number of components may be employed for a given implementation.

For example, the apparatus 30 may be embodied as, or in, a device or apparatus, such as a server, workstation, or mobile device. Examples of the mobile device may include, but are not limited to, tablet computer, laptops, mobile phone, etc. The apparatus 30 may comprise one or more microprocessors or computer processors, which execute appropriate software. The one or more processors 34 of the apparatus 30 may be embodied by one or more of these processors. The software may have been downloaded and/or stored in a corresponding memory, e.g., a volatile memory such as RAM or a nonvolatile memory such as flash. The software may comprise instructions configuring the one or more processors to perform the functions described herein.

It is noted that the apparatus 30 may be implemented as a combination of dedicated hardware to perform some functions and one or more processors (e.g., one or more programmed microprocessors and associated circuitry) to perform other functions. For example, the functional units of the apparatus 30, e.g., sensor interface circuit 32, one or more processors 34, and output circuit 36, may be implemented in the device or apparatus in the form of a logic unit, or a programmable logic, e.g., as a Field-Programmable Gate Array (FPGA). The sensor interface circuit 32 and the output circuit 36 may be implemented by respective interfaces of the apparatus. In general, each functional unit of the apparatus may be implemented in the form of a circuit.

The apparatus 30 may be implemented in several ways. In some examples, the apparatus 30 may be an integral (e.g., designed-in) part of the electrical system 10. Alternatively, the apparatus 30 may only be a separate part of the system. The apparatus 30 may even be retrofitted. For instance, the apparatus 30 may be designed as an independent sensor system that has e.g., its own power supply and own communication interface via which the determined origin of the electrical arc is outputted. In one example, the apparatus 30 may be provided in the form of a mobile device, such as laptop, tablet computer, etc.

As shown in Fig. 2, the sensor interface circuit 34 of the apparatus 30 may include hardware and/or software to enable the apparatus 30 to receive sensor data from the sensor 22a via a wired connection or via a wireless connection. The sensor interface circuit 34 provides the received sensor data to the one or more processors 34.

The one or more processors 34 of the apparatus 30 may execute instructions to perform the method described herein. As described hereinbefore, the one or more processors 34 are configured to analyze the received signal, which includes the sensor data, to determine a direction of the electrical current. Based on the direction of the electrical current, the one or more processors 34 are configured to determine an origin of the electric arc in the electrical system. This will be further described with respect to the examples shown in Figs. 4 and 5.

The output circuit 36 of the apparatus 30 may include hardware and/or software to enable the apparatus 30 to communicate with other electronic devices (e.g. display, storage devices, etc.) and/or a network (LTE, LAN, Wireless LAN, etc.) to provide information relating to the detected arcing event, such as the origin of the arcing event.

Although the example illustrated in Fig. 2 includes a single sensor 22a, it will be appreciated that a plurality of sensors 22 may be provided in some implementations. The plurality of sensors 22 may be arranged in two or more positions in the electrical system 10 for detecting the direction of the electrical current at the corresponding position. In this case, the apparatus 30 may receive, via the sensor interface circuit, at a corresponding signal originating from each of these sensors. The one or more processors 34 of the apparatus 30 may be configured to determine the origin of the electric arc by comparing the at least two received signals.

Fig. 4 illustrates a further example of a system 10 according to an example of the present disclosure. The example system 10 shown in Fig. 4 is similar to the example system 10 shown in Fig. 2, with the exception that an additional sensor 22, such as sensor 22b illustrated in Fig. 4, is provided. Items in Fig. 4 with the same label as in Fig. 2 refer to the same items, and the description of the same item will be not repeated.

The sensor 22b in the example shown in Fig. 4 is removably mounted at the tube-cable interface 18 to monitor the electrical current at this position. As described hereinbefore, during normal operation of the components in Fig. 4, the electrical current flows to the X-ray tube 12. In case of a electric arc event, such as the electric arc 24b shown in Fig. 4 in the cable 14, charge stored in the X-ray tube 12 will flow towards the origin of the electric arc 24b. This results in a reversed current direction at the tube-cable interface 18. Therefore, a straightforward current sign detector may be implemented as the sensor 22b. By employing two sensors 22a and 22b in different positions, as in this example, an electric arc event 24b in the cable can be distinguished from an electric arc event 24a close to or in the HV generator. As mentioned above, in case of an electric arc 24b in the cable, charges stored in the tube will flow towards the arc, resulting in a reversed current direction at the tube-cable interface 18, which can be detected by the sensor 22b. On the other hand, in case of an electric arc event, such as electric arc even shown in Fig. 2, close to or in the HV generator 16, both current detectors 22a and 22b will detect a change in the direction, i.e. the sign, or the current. Therefore, if both sensors 22a and 22b detect a changed sign, the apparatus 10 can determine that an electric arc event occurs close to or in the HV generator 16. On the other hand, if only the sensor 22b detects a changed sign, the apparatus 10 can determine that an electric arc event has occurred in the cable.

Fig. 5 illustrates a further example of a system 10 according to an example of the present disclosure. The example system 10 shown in Fig. 5 is similar to the example system 10 shown in Fig. 4, with the exception that a further sensor 22, such as sensor 22c illustrated in Fig. 5, is provided. Items in Fig. 5 with the same label as in Fig. 4 refer to the same items, and the description of the same item will be not repeated.

As described hereinbefore, the sensors 22a and 22b may be straightforward current direction, or sign, detectors for locating an origin of an electric arc event in the cable 14 and in the HV generator 16. However, in case of an electric arc event 24, such as the electric arc event 24c in the X-ray tube 12, the direction of the electrical current does not change. Only the magnitude of the electrical current changes. Therefore, in order to locate the origin of the electric arc event in the X-ray tube 12, such as electric arc 24c, a further sensor 22, such as sensor 22c, is provided. The sensor 22c may be removably mounted at the tube-cable interface 18 to monitor a magnitude of the electrical current to determine whether an electric arc occurs in the X-ray tube. The sensors 22a, 22b, and 22c provide the acquired sensor data to the apparatus 30 via a wired connection or via a wireless connection. If the sensor 22c detects a change of magnitude of the electrical current and the sensors 22a and 22b do not detect any changed sign, the apparatus 30 can determine that an electric arc 24, such as the electric arc 24c, occurs in the X-ray tube. If both sensors 22a and 22b detect a changed sign, the apparatus 10 can determine whether an electric arc event has occurred close to, or in, the HV generator 16. On the other hand, if only sensor 22b detects a change in the direction, or the sign, of the current, the apparatus 10 can determine that an electric arc event has occurred in the cable 14. Therefore, as illustrated by this example, a combination of straightforward current sign detectors, such as sensors 22a and 22b, with a current magnitude detector, such as sensor 22c, may enable locating an origin of an arcing event in any one of the cable, or the generator, or the X-ray tube in the illustrated generator-cable-tube system 10.

As mentioned above, the magnitude of the electrical current that is detected by each of the sensors, may be used in combination with the detected direction of the electrical current, to determine the hierarchical position of the arcing component within a network of electrical components, and thereby localize the position of the arc with greater accuracy. For instance, the magnitude can be indicative of the position of an arc along a cable. If multiple sensors are provided, the detection of a current having a relatively higher magnitude at one sensor may be deemed to indicate that the origin of the electric arc is closer to that sensor than a sensor that detects a current having a relatively lower magnitude. Similarly, knowledge of the position of the sensors within the network, and the potential origins of the arcing event, may be used to model where an arc is likely to have occurred based on the detected magnitudes of the currents.

Due to the low power consumption of the above-described apparatus 30, a long-lifetime battery mode operation, or energy harvesting may be used to power the apparatus. This obviates the need to supply external power to the sensors 22, such as sensors 22a, 22b, and 22c shown in Figs. 2, 4 and 5. For instance, some energy might be supplied by the power supply to the filament of the X-ray tube, or alternatively some energy might be supplied by the cooling pump supply.
As a further example, in case the X-ray tube is a bipolar X-ray tube, two sensors, such as two current sign detectors, may also be used to detect the current sign of each of the bipolar power supply terminals to the X-ray tube. The information provided by the two current sign detectors may be used to differentiate between effects on the Anode of the X-ray tube, versus the Ground "GND", or to differentiate between effects on the Cathode versus Ground "GND" at the X-ray tube side versus the HV generator side. In this way, an arcing event between the Anode and the Cathode of the X-ray tube may be distinguished from an arcing event between the Anode and Ground "GND", and between the Cathode and Ground "GND". This information may be useful in the correlation of arc data with tube usage, which thereby support root cause finding, and potential arc prevention actions.

Similar to the bipolar X-ray tube example described above, localization of electric arcs in a system with an HV generator consisting of e.g., two power blocks may also profit from at least two straightforward current sign detectors.

In some examples, the integral of the reversed current, i.e., discharging current, can be tracked, e.g., by the sensor 22c shown in Fig. 5, as a measure of arc strength or severity which can be related to the damage that the electric arc caused. Potentially, it can also give additional clues to where the electric arc happened more precisely if tube voltage and stray capacitances are known. This may be valuable e.g., for discrimination between arcing in the Cathode side HV connector versus arcing Cathode-Ground "GND" in the X-ray tube vacuum.

In some examples, at least two sensors (e.g., current, voltage sensors) may be used to provide redundancy, or backup. Rules may also be set in which the origin of an arcing event is assigned to a predetermined position based on the currents measured by the at least two sensors. The mean current from the at least two sensors, or the current with the largest magnitude, may be used to determine, based on the rules, the origin of the arcing event. In case the currents detected by the at least two sensors differ by more than a predetermined threshold, the sensors may be re-calibrated, or it may be determined that a sensor is malfunctioning. Both sensors may be sampled by nearby microcontroller or optical transformer, for example.

In some implementations, the apparatus 30 may store information representing detected events locally for readout at a later point in time. In some implementations, as shown in Fig. 5, the apparatus 30 may communicate a network (e.g., LTE, LAN, Wireless LAN, etc.) via the output circuit 36 to provide information relating to the detected arcing event to a monitoring device 40 for a local or remote analysis. Examples of information relating to the detected arcing event may include, but is not limited to, possible component(s) that require verification, or repair, or replacement; intermediate results of data processing; and raw data from the sensor(s).

The detection of an arcing event. or arcing events, may be used to trigger various service actions. The service actions may mitigate the impact of the electric arc. In some cases, operation of the X-ray tube may be inhibited. In some examples, a remote service application may be executed in the monitoring device 40 for cloud-based service work order generation. The cloud-based service work order generation may be generated based on the sensor-based fault information provided by the apparatus 30. The service work order may comprise automatic service scheduling for a field service engineer, service team, etc.. For example, the service work order may comprise automatically ordering a replacement for a component that is deemed to be close to failure. In some examples, the service work order may comprise a proposal to adapt a hospital's workflow in response to the detected acting event. A preventive maintenance schedule may be recommended in order to reduce the risk of down-time of the component. This may help to reduce the downtime of the medical imaging device.

In some examples, minor arcing events that are not immediately detrimental to the X-ray tube may simply be counted in order to collect statistical information on arcing. *Binning the detected arcing events into two or more types, e.g., minor, medium, and strong, depending on the* magnitude of the change in electrical current *in relation to defined thresholds may be performed in order to provide such statistical information. Correlation of these statistical data with operation modes, e.g. tube voltage, tube age, etc., may provide clues on the root cause of an electric arc, and therefore how arcing may be prevented in future.*

Fig. 6 illustrates an example of a sensor kit 50 for use in detecting an electric arc in an electrical system, such as the electrical system 10 shown in Figs. 2, 4 and 5. The example sensor kit 50 comprises a plurality of sensors 22 for monitoring electrical current. For example, as shown in Fig. 6, the exemplary sensor kit 50 comprises sensors 22a, 22b, 22c, ..., 22n, where n is an integer that is greater than or equal to 2. In some examples, the sensor kit 50 may comprise one or more current sign detectors, such as a resistor, a capacitively or inductively coupled component, and a Hall sensor. In some examples, the sensor kit 50 may comprise one or more current sensors for measuring a magnitude of the electrical current. In some examples, the sensor kit 50 may comprise both a current sign detector(s) and a sensor(s) for measuring a magnitude of the electrical current.

In some examples, some sensors 22 in the sensor kit 50 may have different sensitivities. A doubling of the sensor sensitivity might help to quantify the arcing events and consequently be used to identify trends or arcing events becoming stronger over time.

In some examples, some sensors 22 in the sensor kit 50 may be configured in accordance with different X-ray tube, HV generator, and cable settings. A tuneable / adaptable selection of the sensors may help to adapt for different tube, generator, and cable settings in a modular way.

Fig. 7 illustrates a flowchart describing an example of a method 200 for detecting an electric arc in an electrical system. The method 200 may be implemented as a device, module or related component in a set of logic instructions stored in a non-transitory machine- or computer-readable storage medium such as random access memory (RAM), read only memory (ROM), programmable ROM (PROM), firmware, flash memory, etc., in configurable logic such as, for example, programmable logic arrays (PLAs), field programmable gate arrays (FPGAs), complex programmable logic devices (CPLDs), in fixed-functionality hardware logic using circuit technology such as, for example, application specific integrated circuit (ASIC), complementary metal oxide semiconductor (CMOS) or transistor-transistor logic (TTL) technology, or any combination thereof. For example, computer program code to carry out operations shown in the method 200 may be written in any combination of one or more programming languages, including an object oriented programming language such as JAVA, SMALLTALK, C++, Python, or the like and conventional procedural programming languages, such as the "C" programming language or similar programming languages. For example, the example method may be implemented as the example apparatus 30 shown in Fig. 2, 4 and 5.

In step 210, the method 200 comprises a step of receiving a signal originating from at least one sensor that is arranged in one or more positions in the electrical system for detecting an electrical current.

For example, the apparatus 30 shown in Fig. 2, 4 and 5 may receive a signal originating from at least one sensor 22, such as sensor 22a, 22b, and 22c, for detecting an electrical current.
In step 220, the method 200 comprises a step of analyzing the received signal to determine a direction of the electrical current.

In step 230, the method 200 comprises a step of determining, based on the direction of the electrical current, an origin of the electric arc in the electrical system.

For example, as described above with respect to Fig. 4, in case of an electric arc 24b in the cable, charges stored in the tube will flow to the arc, resulting in a reversed current direction at the tube-cable interface 18, which can be detected by the sensor 22b. On the other hand, in case of an electric arc event, such as electric arc even shown in Fig. 2, close to or in the HV generator 16, both current detectors 22a and 22b will detect a changed sign. Therefore, if both sensors 22a and 22b detect a changed sign, the apparatus 10 can determine that an electric arc event occurs close to or in the HV generator 16. On the other hand, if only sensor 22b detects a changed sign, the apparatus 10 can determine that an electric arc event occurs in the cable.

Optionally, the method 200 may comprise a step of determining a magnitude of an electrical current based on the received signal, and determining the origin of the electric arc in the electrical system based on the determined magnitude of the electrical current in addition to the direction of the electrical current. For example, as described with respect to Fig. 5, if the sensor 22c detects a change of magnitude of the electrical current and the sensors 22a and 22b do not detect any changed sign, the apparatus 30 can determine that an electric arc 24, such as the electric arc 24c, occurs in the X-ray tube. If both sensors 22a and 22b detect a changed sign, the apparatus 10 can determine that an electric arc event occurs close to or in the HV generator 16. On the other hand, if only sensor 22b detects a changed sign, the apparatus 10 can determine that an electric arc event occurs in the cable 14. Therefore, a combination of straightforward current sign detectors, such as sensors 22a and 22b, with a current magnitude detector, such as sensor 22c, may enable locating an origin of an arcing event in all components in the illustrated generator-cable-tube system 10.

Optionally, the integral of the reversed current, i.e., discharging current, may be tracked, e.g., by the sensor 22c shown in Fig. 5, as a measure of arc strength or severity which can be related to the damage that the electric arc caused. Potentially, it can also give additional clues to where the electric arc happened more precisely if tube voltage and stray capacitances are known. This may be valuable e.g., for discrimination between arcing in the cathode side HV connector versus arcing cathode-GND in the tube vacuum. The method 200 may further comprise a step of determining the origin of the electric arc in a particular component, e.g., X-ray tube, based on the integral of the reversed current.

In another example of the present disclosure, a computer program or a computer program element is provided that is characterized by being adapted to execute the method steps of the method according to one of the preceding examples, on an appropriate system.

The computer program element might therefore be stored on a computer unit, which might also be part of an example of the present disclosure. This computing unit may be adapted to perform or induce a performing of the steps of the method described above. Moreover, it may be adapted to operate the components of the above described apparatus. The computing unit can be adapted to operate automatically and/or to execute the orders of a user. A computer program may be loaded into a working memory of a data processor. The data processor may thus be equipped to carry out the method of the disclosure.

This example of the disclosure covers both, a computer program that right from the beginning uses the disclosure and a computer program that by means of an up-date turns an existing program into a program that uses the disclosure.

Further on, the computer program element might be able to provide all necessary steps to fulfil the procedure of an example of the method as described above.

According to a further example of the present disclosure, a computer readable medium, such as a CD-ROM, is presented wherein the computer readable medium has a computer program element stored on it which computer program element is described by the preceding section.

A computer program may be stored and/or distributed on a suitable medium, such as an optical storage medium or a solid state medium supplied together with or as part of other hardware, but may also be distributed in other forms, such as via the internet or other wired or wireless telecommunication systems.

However, the computer program may also be presented over a network like the World Wide Web and can be downloaded into the working memory of a data processor from such a network. According to a further example of the present disclosure, a medium for making a computer program element available for downloading is provided, which computer program element is arranged to perform a method according to one of the previously described examples of the disclosure.

As mentioned above, another aspect of the present disclosure relates to a monitoring apparatus for determining an origin of an arcing event in an X-ray imaging system. Arcing events can occur in different components of an X-ray imaging system, as described above. For instance, arcing events may occur in the voltage generator, in the X-ray tube, or in a cable coupling the X-ray tube to the generator. Arcing events can also occur within different media in such components. For instance, arcing events may occur within media such as vacuum, air, cooling fluid, oil, vacuum, in such components. Arcing events also emit energy in the form of different types of radiation. For instance, arcing events may emit energy in the form of radiofrequency "RF" radiation, (ultra)sonic radiation, and optical radiation. Some of these types of emitted radiation, such as acoustic radiation, can also give rise to vibration when they interact with matter. The inventors have observed that the medium in which an arcing event occurs, and also any shielding that may surround the medium, affect the amount of the different types of radiation that may be detected following an arcing event. Thus, the origin of an arcing event may be determined by detecting different types of radiation that are emitted by an arcing event.

In this regard, a monitoring apparatus 60 for determining an origin of an arcing event 24d in an X-ray imaging system comprising an X-ray tube 12 having a vacuum-containing envelope 62, a liquid cooling circuit 64 for cooling the X-ray tube, and voltage generator 16 configured to power the X-ray tube, is provided. The monitoring apparatus 60 comprises:
a plurality of sensors 66₁, 66₂; and
one or more processors 34;
wherein the plurality of sensors comprises a first sensor 66₁ configured to detect radiofrequency, RF, radiation generated by the arcing event, and a second sensor 66₂ configured to detect vibration and/or acoustic radiation generated by the arcing event; and
wherein the one or more processors 34 are configured to:
   receive signals generated by the first sensor 66₁ and the second sensor 66₂ in response to the detection of the arcing event; and
   selectively identify the origin of the arcing event as one of: within the vacuum-containing envelope 62, within the liquid cooling circuit 64, and within the voltage generator 16, based on the received signals.

This aspect is described with reference to Fig. 8 - Fig. 11, and is based on the observation that signals that the origin of an arcing event may be assigned to one of: within the vacuum-containing envelope 62, within the liquid cooling circuit 64, and within the voltage generator 16, based on the detection of RF radiation generated by the arcing event, and vibration and/or acoustic radiation generated by the arcing event.

Table 1 below summarises various observations relating to the magnitude of detected RF radiation, and vibration and/or acoustic radiation, in response to arcing events with different origins in an X-ray imaging system.

**Table 1**

| | **Detection of RF radiation** | **Detection of vibration and/or acoustic radiation** |
|---|---|---|
| **Arc origin within vacuum-containing envelope** | Strong | Weak/ indetectable |
| **Arc origin within liquid cooling circuit** | Strong | Strong |
| **Arc origin within voltage generator** | Weak/ indetectable | Strong |

As illustrated in Table 1, an arc that has been generated within the vacuum-containing envelope 62, typically generates relatively strongly-detectable RF radiation, and relatively weakly-detectable, or undetectable, vibration and/or acoustic radiation. By contrast, an arc that has been generated within the liquid cooling circuit 64, typically generates relatively strongly-detectable RF radiation as well as relatively strongly-detectable vibration and/or acoustic radiation. By contrast, an arc that has been generated within the voltage generator typically generates relatively weakly-detectable, or undetectable, RF radiation, and relatively strongly-detectable vibration and/or acoustic radiation. This is due to the inability of the vacuum-containing envelope 62 to transmit vibration and/or acoustic radiation, and the high degree of RF shielding provided by the RF generator, and which consequently reduce the transmission of vibration and/or acoustic radiation, and RF radiation, respectively. Thus, as may be appreciated from Table 1, by measuring both RF radiation, and vibration and/or acoustic radiation, that are emitted by an arcing event, the origin of the arcing event may be selectively identified as one of: within the vacuum-containing envelope 62 within the liquid cooling circuit 64, and within the voltage generator 16.

The ability to determine the origin of an arcing event facilitates the verification, repair, or replacement of the affected component in a time-efficient manner, as described above. Moreover, the ability to distinguish the origin of an arcing event between the vacuum-containing envelope, the liquid cooling circuit, and the voltage generator may facilitate a repair of a component rather than its entire replacement. For instance if the origin of an arcing event is determined to be within the liquid cooling circuit, the cooling liquid may be replaced rather than replacing the entire X-ray tube. Likewise, the origin of an arcing event is determined to be within the vacuum-containing envelope, a repair may be possible by "re-conditioning" the X-ray tube, or performing a "tube degassing" procedure on the X-ray tube, rather than replacing it.

Fig. 8 is a schematic diagram illustrating a first example of a monitoring apparatus 60 for determining an origin of an arcing event 24d in an X-ray imaging system, in accordance with some aspects of the present disclosure. With reference to Fig. 8, a first sensor 66₁ is configured to detect radiofrequency, RF, radiation generated by the arcing event, and a second sensor 66₂ configured to detect vibration and/or acoustic radiation generated by the arcing event.

The first sensor 66₁ illustrated in Fig. 8 may be provided in the form of an antenna, as illustrated in Fig. 8. Various types of antennae may be used for this purpose, including a dipole antenna, a stripline antenna, a coil antenna, and so forth. A directional antenna may also be used to provide relatively higher sensitivity towards a desired direction, and consequently component in the X-ray imaging system. The size, and type of the antenna may be selected based on its frequency response. In some cases, arcs originating in the vacuum-containing envelope have been observed with frequencies in excess of 500 MHz. The first sensor 66₁ may also include a sensor interface circuit (not shown in Fig. 8). The sensor interface circuit may include an amplifier and/or a detection circuit and which are used to respectively amplify, and convert the detected RF radiation into signals that are then inputted into the one or more processors 34. The sensor interface circuit may include a filter configured to filter-out signals with frequencies outside a desired range of interest. The sensor interface circuit may include a digital-to-analogue converter "DAC" to digitise the signals prior to their inputting into the one or more processors 34. The signals generated by the first sensor 66₁ may be communicated to the one or more processors 34 via any form of data communication, including via wired, wireless, and optical communication.

The second sensor 66₂ illustrated in Fig. 8 may be provided in the form of a microphone, or a vibration sensor. The second sensor 66₂ may also include a sensor interface circuit (not shown in Fig. 8). The sensor interface circuit may include an amplifier and/or a detection circuit and which are used to respectively amplify, and convert the detected vibration and/or acoustic radiation into signals that are then inputted into the one or more processors 34. The sensor interface circuit may include a filter configured to filter-out signals with frequencies outside a desired range of interest. The sensor interface circuit may include a digital-to-analogue converter "DAC" to digitise the signals prior to their inputting into the one or more processors 34. The signals generated by the second sensor 66₂ may be communicated to the one or more processors 34 via any form of data communication, including via wired, wireless, and optical communication.

The first sensor 66₁ and the second sensor 66₂ illustrated in Fig. 8 may be arranged in the vicinity of the X-ray tube. The first sensor 66₁ and the second sensor 66₂ may be arranged so as to provide a direct, uninterrupted, path to the X-ray tube. For instance, it may be arranged that there is no, or minimal, acoustic or RF shielding between the sensor and the X-ray tube. If the second sensor 66₂ is provided in the form of a vibration sensor, the vibration sensor may be mechanically coupled to the X-ray tube. The vibration sensor may be directly or indirectly coupled to the X-ray tube. For instance, the vibration sensor may be coupled to a casing of the X-ray tube, or it may be coupled to the gantry of a CT imaging system in which the X-ray tube is used, or to an element in the liquid cooling circuit of the X-ray tube such as a pump or a fluidic connector, for example.

In this example, the one or more processors may selectively identify the origin of the arcing event as one of: within the vacuum-containing envelope 62, within the liquid cooling circuit 64, and within the voltage generator 16, based on the received signals, by determining the magnitude of the detected signals and applying the decision logic described in Table 1. In order to facilitate the application of this decision logic, thresholds may be applied to the detected signals in order to determine whether they represent "weak/ undetectable signals", or "strong" signals. The thresholds depend on the relative location of the sensor to the origin of the arcing event. The thresholds may be set based on experimentally-measured levels, or based on modelled levels of the signals generated by an arcing event.

In a related example, the one or more processors 34 of the monitoring apparatus 60 are further configured to:
perform a spectral analysis on the signals generated by the first sensor 66₁ and the second sensor 66₂ to generate corresponding first and second frequency spectra;
determine a similarity between each of the first and second frequency spectra and one or more reference frequency spectra, the reference frequency spectra representing signals generated within one or more of: the vacuum-containing envelope 62, the liquid cooling circuit 64, and the voltage generator 16, by the first sensor 66₁ and the second sensor 66₂, respectively; and
wherein the one or more processors 34 are configured to selectively identify the origin of the arcing event, based further on the similarity.

This example is based on the observation that RF radiation generated by the arcing event, and vibration and/or acoustic radiation generated by the arcing event, have characteristic signatures that may be used to identify their origin For instance, the RF spectrum generated in response to an arcing event within the vacuum-containing envelope 62 is different to the RF spectrum generated in response to an arcing event within the liquid cooling circuit 64. Thus, by performing a spectral analysis on the signals generated by the first sensor 66₁ and the second sensor 66₂ to generate corresponding first and second frequency spectra, the frequency spectra may be compared with reference frequency spectra that are characteristic of signals generated by the first sensor 66₁ and the second sensor 66₁, respectively. The reference frequency spectra may be generated within e.g. the vacuum-containing envelope 62, the liquid cooling circuit 64, and the voltage generator 16. If the detected spectrum is similar to a reference spectrum, the origin of the reference spectrum may be assigned to the detected spectrum. This provides increased confidence in the origin of the arcing event.

In this example, the spectral analysis may include performing a Fourier transform on the signals generated by the first sensor 66₁ and the second sensor 66₂ to generate corresponding first and second frequency spectra. This may be implemented by the one or more processors 34 using known signal processing techniques. The reference frequency spectra representing signals generated within the vacuum-containing envelope 62, the liquid cooling circuit 64, and the voltage generator 16, for each of the first sensor 66₁ and the second sensor 66₁, may be generated from empirical measurements of such signals. For instance, signals may be recorded over a period of time that is sufficient to capture an arcing event. After performing a retrospective analysis of the X-ray tube to determine the origin of the arcing event, the spectra may be labelled as characteristic of an arcing event in this specific origin. The one or more processors may determine a measure of the similarity between each of the first and second frequency spectra and the reference spectra by performing a cross-correlation between the spectra, for example. If the result of determining the similarity between the spectra is above a predetermined threshold, the origin of the arcing event may be attributed to this origin. Thus, this example provides confirmatory, or more detailed, information on the origin of the arcing event, and thereby provides increased confidence in the determined origin of the arcing event.

In another related example, the plurality of sensors of the monitoring apparatus 60 comprises a third sensor 66₃ configured to detect radiofrequency, RF, radiation generated by the arcing event, and a fourth sensor 66₄ configured to detect vibration and/or acoustic radiation generated by the arcing event. In this example, the one or more processors 34 are further configured to:
receive signals generated by the third sensor 66₃ and the fourth sensor 66₄ in response to the detection of the arcing event; and
estimate a position of the origin of the arcing event based on the detected RF radiation generated by the arcing event using the signals generated by the first sensor 66₁ and the third sensor 66₃; and
estimate a position of the origin of the arcing event based on the detected vibration and/or acoustic radiation generated by the arcing event using the signals generated by the second sensor 66₂ and the fourth sensor 66₄; and
wherein the one or more processors 34 are configured to selectively identify the origin of the arcing event based further on the position of the origin of the arcing event estimated based on the detected RF radiation and/or the position of the origin of the arcing event estimated based on the detected vibration and/or acoustic radiation.

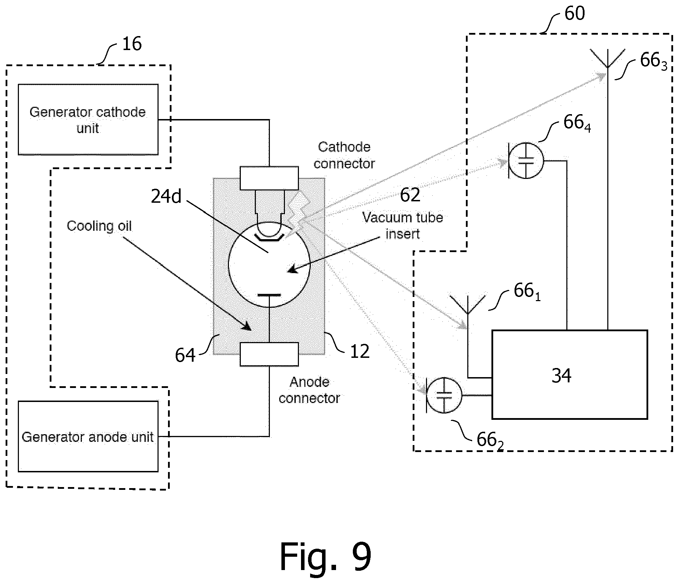

This example is described with reference to Fig. 9, which is a schematic diagram illustrating a second example of a monitoring apparatus 60 for determining an origin of an arcing event 24d in an X-ray imaging system, in accordance with some aspects of the present disclosure. The items illustrated in Fig. 9 that have the same labels as in Fig. 8 refer to the same item, and a description of the item is not duplicated for sake of brevity. In addition to the items illustrated in Fig. 8, the example illustrated in Fig. 9 also includes a third sensor 66₃ and a fourth sensor 66₄.

In this example, the signals generated by the third sensor 66₃ and the fourth sensor 66₄ are therefore used in combination with the signals generated by the first sensor 66₁ and the second sensor 66₂, respectively, to determine the origin of the arcing event. In this example, the operations of estimating a position of the origin of the arcing event, may in general be performed by comparing the times, or the phases, or the magnitudes, of the signals detected by the first sensor 66₁ and the third sensor 66₃, and likewise comparing the times, or the phases, or the magnitudes, of the signals detected by the second sensor 66₂ and the fourth sensor 66₄. For instance, in one technique the times of the detected signals may be compared in order to determine that the origin of the arcing event is nearer to the sensor that generated the earlier signal in response to the arcing event. Similarly, in another technique, the magnitudes of the detected signals may be compared in order to determine that the origin of the arcing event is nearer to the sensor that generated the signal with the relatively larger magnitude in response to the arcing event. Similarly, in another technique, the phases of the detected signals may be compared in order to determine that the origin of the arcing event is nearer to the sensor that generated the signal with a relatively earlier specified phase angle in response to the arcing event.

In this example, the times of the signals detected by sensors of the same type, i.e. the first sensor 66₁ and the third sensor 66₃, or the second sensor 66₂ and the fourth sensor 66₄, may be determined by applying a threshold to the detected signals and measuring the time at which the threshold is reached. As described above, the relative times of the detected signals may be used to determine that the origin of the arcing event is nearer to the sensor that detected the radiation first. Similarly, the magnitudes, or the phases, of the detected signals may be determined and used to simply determine that the origin of the arcing event is nearer to the sensor that detected the radiation with a greater magnitude, or with an earlier phase. The phase, in this example, may be determined by including a phase detector in the sensor interface circuit(s) described above.

In another technique, a triangulation operation is performed. In this technique, the operation of estimating a position of the origin of the arcing event based on the detected RF radiation generated by the arcing event, may include performing a triangulation operation on the signals generated by the first sensor 66₁ and the third sensor 66₃ to provide the estimated position of the origin of the arcing event based on the detected RF radiation. The operation of estimating a position of the origin of the arcing event based on the detected RF radiation generated by the arcing event, may include performing a triangulation operation on the signals generated by the second sensor 66₂ and the fourth sensor 66₄ to provide the estimated a position of the origin of the arcing event based on the detected vibration and/or acoustic radiation.

The triangulation may be performed by comparing the times, or the phases, or the magnitudes, of the signals detected by the first sensor 66₁ and the third sensor 66₃, and likewise comparing the times, or the phases, or the magnitudes, of the signals detected by the second sensor 66₂ and the fourth sensor 66₄. By providing two sensors that detect each type of radiation, e.g. RF radiation, or vibration and/or acoustic radiation, in accordance with this technique, the one or more processors may perform a triangulation operation that estimates the position of the origin of the arcing event as a position anywhere along an arc of revolution around an axis connecting the two sensors. By providing three sensors that detect each type of radiation, the one or more processors may perform a triangulation operation that estimates the position of the origin of the arcing event as a point in space. This assumes that the three sensors are arranged in a non-collinear manner.

The additional spatial information on the origin of the arcing event that is provided in accordance with this example provides increased confidence in the determined origin.

If triangulation is used with the signals generated by the second sensor 66₂ and the fourth sensor 66₄, the time of detecting the RF radiation with the first sensor 66₁ or the second sensor 66₂, may serve as an additional timestamp from which the path lengths for the vibration/ sound radiation travelling between the origin of the arcing event and the second and fourth sensors 66₂, 66₄, may be calculated. Since vibration/ sound travels more slowly than RF radiation, if RF radiation is detected by the first and/or third sensors 66₁, 66₃, the time of detecting the RF radiation with these sensors may serve as an additional timestamp from which the path lengths for the vibration/ sound radiation travelling between the origin of the arcing event and the second and fourth sensors 66₂, 66₄, may be calculated.

In another related example, the monitoring apparatus 60 comprises at least one current sensor 68₁, 68₂. The at least one current sensor 68₁, 68₂ is configured to detect an electrical current flowing into or returning from at least one of: the X-ray tube, the voltage generator, and an electrical cable 70₁, 70₂ coupling the X-ray tube to the voltage generator. The one or more processors 34 are further configured to receive signals generated by the at least one current sensor 68₁, 68₂ in response the arcing event. The one or more processors 34 are configured to selectively identify the origin of the arcing event based further on the signals generated by the at least one current sensor 68₁, 68₂.

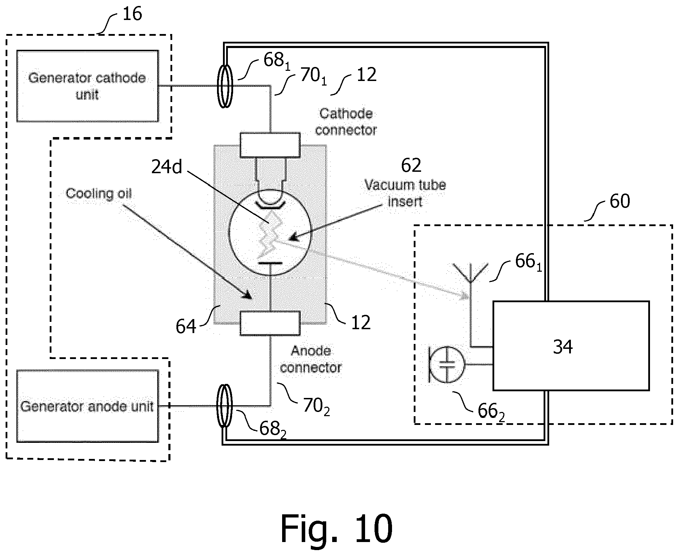

This example is described with reference to Fig. 10, which is a schematic diagram illustrating a third example of a monitoring apparatus 60 for determining an origin of an arcing event 24d in an X-ray imaging system, in accordance with some aspects of the present disclosure. The items illustrated in Fig. 10 that have the same labels as in Fig. 8 refer to the same item, and a description of the item is not duplicated for sake of brevity. In addition to the items illustrated in Fig. 8, the example illustrated in Fig. 10 also includes current sensors 68₁, and 68₂. The current sensor(s) in this example detect electrical currents that flow into, or that return from, one or more of: the X-ray tube, the voltage generator, and the electrical cable 70₁, 70₂. In the illustrated example, two current sensors are illustrated. The current sensor 68₁ is configured to detect an electrical current returning from the X-ray tube. The current sensor 68₂ is configured to detect an electrical current flowing into the X-ray tube. In general there may be one or more current sensors. Electrical arcing events can give rise to perturbations (e.g. spikes) in such detected currents, depending on the origin of the arcing event. For instance, an arcing event originating within the vacuum-containing envelope of the X-ray tube can give rise to a relatively larger perturbation in an electrical current flowing into or returning from the X-ray tube, than an electrical arcing event originating within the liquid cooling circuit. Thus, the additional information that is provided by the detected electrical currents may be used to identify the origin of the arcing event with increased confidence. The current sensor may also be used to selectively identify the origin of the arcing event as within the electrical cable 70₁, 70₂, based on the received signals generated by the at least one current sensor 68₁, 68₂. For instance, an arcing event originating within the cable may give rise to a perturbation with a smaller magnitude than an arcing event originating within the X-ray tube.

In this example, the at least one current sensor 68₁, 68₂, may be provided by a coil. The coil may be wrapped around or located adjacent to the relevant conductor in which the current is to be measured. The former situation is illustrated in Fig. 10. Such a coil may also be referred-to as a Rogowski coil. A Rogowski coil does not include a metal core. However, a current sensor that is provided in the form of a coil may also include a metal (typically iron-based) core. Other types of current sensors may be used as current sensor 68₁, 68₂, as described above. For instance, the current sensor 68₁, 68₂ may include a resistor, and wherein the current passing through the resistor is determined by measuring a voltage drop across the resistor. Other types of current sensors may be used, including capacitively- or inductively-coupled current sensors, and a Hall sensor, as described above.

In this example, the one or more processors 34 may be configured to selectively identify the origin of the arcing event based further on the signals generated by the at least one current sensor 68₁, 68₂, using various techniques. For instance, in some examples, the presence of a current at the position of the current sensor 68₁, 68₂ is sufficient to confirm that arcing has occurred at, or close to, the position of the measured current. For instance, if the magnitude of a current spike that is detected using the current sensor exceeds a predetermined threshold, the current spike may be deemed to be indicative of an arcing event at, or close to, the position of the measured current spike. In another example, multiple current sensors are used, and the detection of a current spike at a position and the absence of a detected current spike at another position, may be deemed to be indicative of an arcing event at one position and not the other.

In another related example, the one or more processors 34 of the monitoring apparatus 60 are configured to determine a direction of the electrical current detected by the at least one current sensor 68₁, 68₂. The one or more processors are configured to selectively identify the origin of the arcing event based further on the direction of the electrical current detected by the at least one current sensor 68₁, 68₂. This example operates in the manner described above with reference to Fig. 1 - Fig. 7. In other words, the direction of the electrical current that is generated in response to the arcing event can indicate which side of the current sensor the arcing event occurred.

In another related example the one or more processors 34 of the monitoring apparatus 60 are further configured to automatically generate a service work order 72 based on the received signals generated by the first sensor and/or the second sensor in response to the detection of the arcing event. The service work order comprises information representing the identified origin of the arcing event.

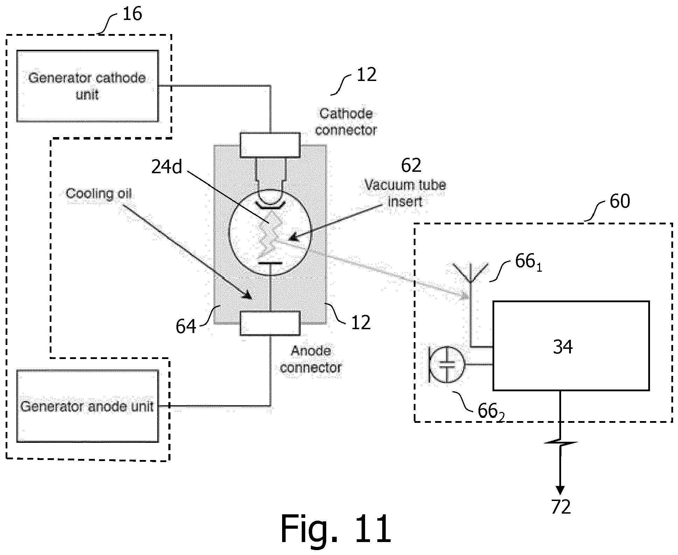

This example is described with reference to Fig. 11, which is a schematic diagram illustrating a fourth example of a monitoring apparatus 60 for determining an origin of an arcing event 24d in an X-ray imaging system, in accordance with some aspects of the present disclosure. The items illustrated in Fig. 11 that have the same labels as in Fig. 8 refer to the same item, and a description of the item is not duplicated for sake of brevity. In addition to the items illustrated in Fig. 8, the example illustrated in Fig. 10 also illustrates the outputting of a service work order 72. The service work order may for instance indicate that an arc has occurred in the vacuum-containing envelope 62. The service work order may also include additional information representing the severity of the arc, the timing of the arc, and so forth. The severity of the arc may be measured based on the magnitude and/or the duration of the signals detected by the first sensor 66₁, and the second sensor 66₂, for example.

Thus, the service work order enables a service engineer to verify the operation of the vacuum-containing envelope 62, the liquid cooling circuit 64, and the voltage generator 16, as necessary, in a time-efficient manner.

In another related example, an X-ray imaging system that includes the monitoring apparatus 60, is provided. The X-ray imaging system may for example be a projection X-ray imaging system such as an X-ray C-arm-based *imaging* system or a digital X-ray radiography "DXR" *imaging* system, or a computed tomography "CT" *imaging* system.

This aspect of the present disclosure relating to a monitoring apparatus is described in the following enumerated Examples:
Example 1. A monitoring apparatus (60) for determining an origin of an arcing event (24d) in an X-ray imaging system comprising an X-ray tube (12) having a vacuum-containing envelope (62), a liquid cooling circuit (64) for cooling the X-ray tube, and voltage generator (16) configured to power the X-ray tube, the monitoring apparatus (60) comprising:
   a plurality of sensors (66₁, 66₂); and
   one or more processors (34);
   wherein the plurality of sensors comprises a first sensor (66₁) configured to detect radiofrequency, RF, radiation generated by the arcing event, and a second sensor (66z) configured to detect vibration and/or acoustic radiation generated by the arcing event; and
   wherein the one or more processors (34) are configured to:
      receive signals generated by the first sensor (66₁) and the second sensor (66₂) in response to the detection of the arcing event; and
      selectively identify the origin of the arcing event as one of: within the vacuum-containing envelope (62), within the liquid cooling circuit (64), and within the voltage generator (16), based on the received signals.
Example 2. The monitoring apparatus according to Example 1, wherein the one or more processors (34) are further configured to:
   perform a spectral analysis on the signals generated by the first sensor (66₁) and the second sensor (66₂) to generate corresponding first and second frequency spectra;
   determine a similarity between each of the first and second frequency spectra and one or more reference frequency spectra, the reference frequency spectra representing signals generated within one or more of: the vacuum-containing envelope (62), the liquid cooling circuit (64), and the voltage generator (16), by the first sensor (66₁) and the second sensor (66₂), respectively; and
   wherein the one or more processors (34) are configured to selectively identify the origin of the arcing event, based further on the similarity.
Example 3. The monitoring apparatus according to Example 1, wherein the plurality of sensors further comprises a third sensor (66₃) configured to detect radiofrequency, RF, radiation generated by the arcing event, and a fourth sensor (66₄) configured to detect vibration and/or acoustic radiation generated by the arcing event; and
   wherein the one or more processors (34) are further configured to:
      receive signals generated by the third sensor (66₃) and the fourth sensor (66₄) in response to the detection of the arcing event;
      estimate a position of the origin of the arcing event based on the detected RF radiation generated by the arcing event using the signals generated by the first sensor (66₁) and the third sensor (66₃); and
      estimate a position of the origin of the arcing event based on the detected vibration and/or acoustic radiation generated by the arcing event using the signals generated by the second sensor (66₂) and the fourth sensor (66₄); and
   wherein the one or more processors (34) are configured to selectively identify the origin of the arcing event based further on the position of the origin of the arcing event estimated based on the detected RF radiation and/or the position of the origin of the arcing event estimated based on the detected vibration and/or acoustic radiation.
Example 4. The monitoring apparatus according to any one of Examples 1-3, further comprising at least one current sensor (68₁, 68₂);
   wherein the at least one current sensor (68₁, 68₂) is configured to detect an electrical current flowing into or returning from at least one of: the X-ray tube, the voltage generator, and an electrical cable (70₁, 70z) coupling the X-ray tube to the voltage generator; and
   wherein the one or more processors (34) are further configured to receive signals generated by the at least one current sensor (68₁, 68₂) in response the arcing event; and
   wherein the one or more processors (34) are configured to selectively identify the origin of the arcing event based further on the signals generated by the at least one current sensor (68₁, 68₂).
Example 5. The monitoring apparatus according to Example 4, wherein the one or more processors (34) are further configured to selectively identify the origin of the arcing event as within the electrical cable (70₁, 70₂), based on the received signals generated by the at least one current sensor (68₁, 68₂).
Example 6. The monitoring apparatus according to Example 4 or Example 5, wherein the one or more processors (34) are configured to determine a direction of the electrical current detected by the at least one current sensor (68₁, 68₂); and
   wherein the one or more processors are configured to selectively identify the origin of the arcing event based further on the direction of the electrical current detected by the at least one current sensor (68₁, 68₂).
Example 7. The monitoring apparatus according to any one of Examples 1-6, wherein the one or more processors (34) are further configured to automatically generate a service work order (72) based on the received signals generated by the first sensor and/or the second sensor in response to the detection of the arcing event; and
   wherein the service work order comprises information representing the identified origin of the arcing event.
Example 8. An X-ray imaging system comprising the monitoring apparatus according to any one of Examples 1-7.
Other variations to the disclosed examples can be understood and effected by those skilled in the art, from a study of the drawings, the disclosure, and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measured cannot be used to advantage. Any reference signs in the claims should not be construed as limiting the scope.

## Claims

1. An apparatus (30) for detecting an electric arc (24a, 24b, 24c) in an electrical system (10), the apparatus comprising:
- a sensor interface circuit (32); and
- one or more processors (34);
wherein the sensor interface circuit is configured to receive a signal originating from at least one sensor (22a, 22b, 22c) that is arranged in one or more positions (18, 20) in the electrical system for detecting an electrical current; and
wherein the one or more processors are configured to analyze the received signal to determine a direction of the electrical current at the one or more positions in the electrical system, and to determine, based on the direction of the electrical current, an origin of the electric arc in the electrical system.

2. The apparatus according to claim 1,
wherein the one or more processors are configured to analyze the received signal to determine a magnitude of the electrical current at one or more positions in the electrical system; and
wherein the one or more processors are configured to determine the origin of the electric arc in the electrical system based further on the magnitude of the electrical current.

3. The apparatus according to claim 1 or 2,
wherein the one or more processors are configured to determine a time period during which the electrical current has reversed its direction at the one or more positions in the electrical system during an operation of the electrical system, to determine an integral of the electrical current over the determined time period, and to determine an arc strength of the electric arc based on the determined integral; and
wherein the one or more processors are configured to determine the origin of the electric arc in the electrical system based further on the magnitude of the electrical current.

4. The apparatus according to any one of the preceding claims,
wherein the one or more processors are configured to compare the measured electrical current with a defined threshold to determine a severity of the electric arc.

5. The apparatus according to claim 4,
wherein in response to determining that the severity of the electric arc exceeds the defined threshold, the one or more processors are configured to trigger an action to mitigate the severity of the electric arc.

6. The apparatus according to claim 4,
wherein in response to determining that the severity of the electric arc is below the defined threshold, the one or more processors are configured to record information relating to the electric arc.

7. The apparatus according to any one of the preceding claims,
wherein the sensor interface circuit is configured to receive at least two signals originating from at least two sensors that are arranged in at least two positions in the electrical system for detecting the electrical current; and
wherein the one or more processors are configured to determine the origin of the electric arc by comparing the at least two received signals.

8. The apparatus according to any one of the preceding claims, further comprising:
- an output circuit (36);
wherein the one or more processors are configured to provide information relating to the electric arc via the output circuit.

9. The apparatus according to any one of the preceding claims,
wherein the electrical system comprises an X-ray tube, a high-voltage generator, and a cable connecting the X-ray tube and the high-voltage generator.

10. The apparatus according to any one of claims 1 to 8,
wherein the electrical system comprises a high-power transmitting radio device.

11. A system (110) for detecting an electric arc (24a, 24b, 24c) in an electrical system (10), the system comprising:
- at least one sensor; and
- an apparatus according to any one of the preceding claims;
wherein the at least one sensor is removably mountable at one or more positions in an electrical system for detecting an electrical current in the electrical system; and
wherein the apparatus is configured to receive a signal originating from the at least one sensor and to detect an electric arc in the electrical system.

12. The system according to claim 11,
wherein the at least one sensor comprises a current sign detector for detecting a direction of the electrical current.

13. The system according to claim 11 or 12, further comprising:
- a monitoring device (40) configured to provide a local or remote analysis of information relating to the electric arc provided by the apparatus.

14. A method (200) for detecting an electric arc (24a, 24b, 24c) in an electrical system (10), the method comprising:
- receiving (210) a signal originating from at least one sensor that is arranged in one or more positions in the electrical system for detecting an electrical current;
- analyzing (220) the received signal to determine a direction of the electrical current at the one or more positions in the electrical system; and
- determining (230), based on the direction of the electrical current, an origin of the electric arc in the electrical system.

15. A sensor kit (50) for use in detecting an electric arc (24a, 24b, 24c) in an electrical system (10), the sensor kit comprising a plurality of sensors (22a..n) for detecting an electrical current.

16. A monitoring apparatus (60) for determining an origin of an arcing event (24d) in an X-ray imaging system comprising an X-ray tube (12) having a vacuum-containing envelope (62), a liquid cooling circuit (64) for cooling the X-ray tube, and voltage generator (16) configured to power the X-ray tube, the monitoring apparatus (60) comprising:
a plurality of sensors (66₁, 66₂); and
one or more processors (34);
wherein the plurality of sensors comprises a first sensor (66₁) configured to detect radiofrequency, RF, radiation generated by the arcing event, and a second sensor (66z) configured to detect vibration and/or acoustic radiation generated by the arcing event; and
wherein the one or more processors (34) are configured to:
receive signals generated by the first sensor (66₁) and the second sensor (66₂) in response to the detection of the arcing event; and
selectively identify the origin of the arcing event as one of: within the vacuum-containing envelope (62), within the liquid cooling circuit (64), and within the voltage generator (16), based on the received signals.
